# EUROPEAN PATENT SPECIFICATION

(11) **EP 1 460 516 B1**
(45) Date of publication and mention of the grant of the patent: **07.05.2008**
(21) Application number: 04014203.6
(22) Date of filing: 30.07.1999
(51) Int. Cl.: G01N 33/487, A61B 5/00, G06F 19/00

(54) **Docking station for receiving a hand-held analyte test instrument**
Ankoppelstation für ein tragbares Analysegerät
Base pour instrument d'analyse portable

(30) Priority: 31.07.1998 US 94895 P
(43) Date of publication of application: 22.09.2004
(62) Divisional of application: 99938895.2
(73) Proprietor: ABBOTT LABORATORIES, Abbott Park, IL 60064-6008 (US)
(72) Inventor: Krishnaswamy, Sarath, Chelmsford, MA 01824 (US); Guiney, Patrick, Concord, MA 01742 (US)
(74) Representative: Modiano, Micaela Nadia

(56) References cited:
- EP-A- 0 290 683
- WO-A-94/24929
- DE-A1- 4 315 532
- US-A- 5 307 263

## Description

### BACKGROUND OF THE INVENTION

Health care professionals at medical institutions are routinely required to use various instruments to perform bedside tests on patients to monitor various aspects of patients' health. These tests generate substantial amounts of medical data which is often collected and organized for subsequent analysis. The data can include results of tests to determine the level of one or more analytes (e.g., blood glucose, ketones).

Traditionally, the primary means for collecting and organizing data obtained from the instruments is a printed or transcribed record of the test results. To review the results, a health care professional either retrieves the results from the institution's records department or goes to the patient's room. Since these results are often available only in printed form, chronological and statistical analysis is difficult.

Government regulations require medical institutions to perform control tests on instruments used for patient testing at regular intervals to ensure the accuracy of test results. Health care professionals that operate such instruments are also required to undergo periodic recertification.

Members of the institution's administrative staff are frequently responsible for the review of instrument control test data and recertification procedures to ensure compliance with federal regulations. In many instances, however, administrators identify tests involving "out-of-specification" instruments, expired supplies (e.g., test strips), or uncertified health care professionals after testing has been completed. These test results are either accepted or the patient can be subjected to another test.

It is therefore desirable to have a health data management system in which each of a plurality of medical test instruments are connected to a data communications network to provide real time transfer of patient test results to a centralized location. In addition, it is desirable to include in such a system a security mechanism for preventing testing of patients with "out-of-specification" instruments, expired supplies (e.g., test strips), or uncertified health care professionals.

EP0290683A discloses an outpatient monitoring system comprising three elements: a patient operated monitor/recorder; a master computer that develops a programme on the basis of the information recorded by the patient; and an interface unit used to transfer data between the recorder and the master computer or a printer or a modem. The recorder with a keyboard operates under the control of a microprocessor connected to memory, and incorporates a blood glucose strip reader and automatically stores the measured values in the memory. The recorder also includes a standard serial interface to interface the system with the interface unit. The recorder simply plugs into the interface unit that in turn plugs into the master computer or modem. Alternatively the data can be printed out on a printer that can be incorporated in the interface unit.

WO-A-94/24929 discloses a patient monitor and support system including a plurality of patient sites individually connected via a set of communications links to a care centre including a plurality of computer workstations. Each patient site is controlled by a base unit in communication to the care centre by means of the communications links, which may comprise radio communications link, a modem/telephone line link, a fibre optic link, and specifically a telephone line to establish communications via a modem provided at the care centre. Portable recorders, that may record inputs from sensors such as reagent strips, may be docked in docking ports of the base unit to upload or download data and to charge the batteries of the recorders.

### SUMMARY OF THE INVENTION

A docking station as defined in claim 1 has been developed.

### BRIEF DESCRIPTION OF THE DRAWINGS

The foregoing and other objects, features and advantages of the invention will become apparent from the following more particular description of preferred embodiments of the invention, as illustrated in the accompanying drawings. The drawings are not necessarily to scale, emphasis instead being placed on illustrating the principles of the present invention.

FIG. 1 is a perspective view of an analyte test instrument disposed in a docking station.

FIG. 2 is a functional block diagram of a plurality of medical test instruments connected to a host computer over a data communications network.

FIGS. 3A - 3C are top, side and end views, respectively, of an analyte test instrument.

FIG. 4 is a cut-away perspective view of an analyte test instrument.

FIG. 5 is a sample of displayed data provided on a LCD module of an analyte test instrument.

FIG. 6 is a perspective view of a three-electrode test strip for use with an analyte test instrument.

FIGS. 7 is a perspective view of the back of an analyte test instrument opened to expose a battery compartment and a separate rechargeable battery pack.

FIGS. 8A - 8B are illustrations of a rechargeable battery pack and a two finger leaf spring contact connector for use with the rechargeable battery pack, respectively.

FIGS. 9A - 9B are cross-sectional views of an analyte test instrument shown with alkaline power cells and with a rechargeable battery pack, respectively.

FIG. 10 is an illustration of a battery monitoring circuit and recharge current circuit employing a pair of two finger leaf spring contact connectors.

FIGS. 11A - 11B is a perspective view of a docking station for use with an analyte test instrument.

FIG. 12 is a functional block diagram of a docking station switching circuit for directing data transfer between two ports.

### DETAILED DESCRIPTION

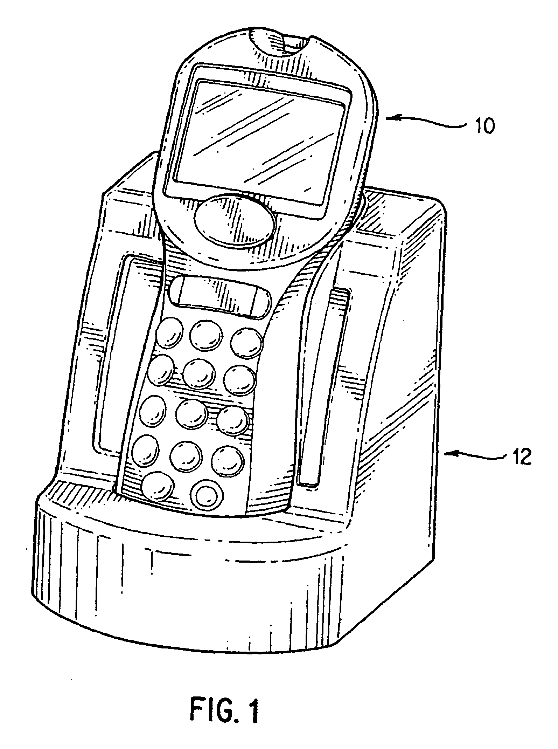

Referring to FIG. 1, an instrument 10 used in patient testing for one or more analytes (e.g., blood glucose, ketones, etc.) in a hospital environment is shown positioned in a docking station 12. The instrument 10 analyzes a patient sample (e.g., blood) deposited on one end of a test strip when the other end of the strip is inserted in the instrument 10. The docking station 12 allows for automatic transfer of test results to a host computer and provides power to recharge an internal battery pack when the instrument 10 is positioned in the station 12.

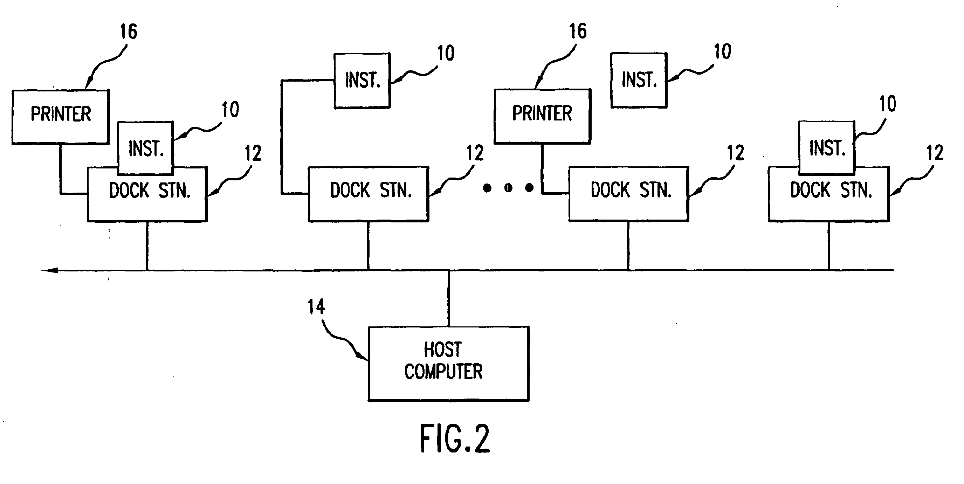

In a typical health care facility, a plurality of instruments 10 can be networked to a host computer 14 though docking stations 12 as shown in FIG. 2. For example, one instrument can be assigned to each patient room. A nurse or other operator inserts a test strip into the instrument and deposits a patient sample onto an exposed portion of the test strip. An audible indicator alerts the operator when a sufficient patient sample volume for analysis has been deposited on the test strip. The instrument then analyzes the sample and displays the results on the LCD module. The operator can return the instrument to the docking station 12, even before the results are available, where the test data (operator ID, patient ID, date, time, and other parameters) are automatically transferred via a cable to the host computer 14. The test data and results can be directed to a local printer 16 by a cable (e.g., an RS-232 standard interface cable) to generate a hardcopy, if desired. The network is controlled by the host computer 14 via a bi-directional data communication link, i.e., data can be transferred from the instrument 10 to the computer 14 and data can be transferred from the computer 14 to the instrument 10. The latter mode allows for remote independent configuration of individual instruments or groups of instruments.

### Analyte Test Instrument

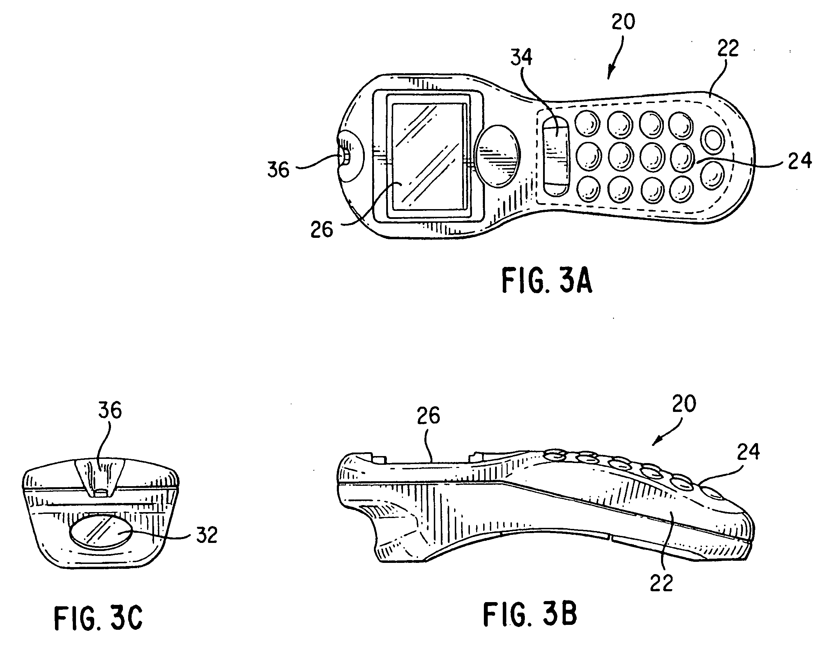

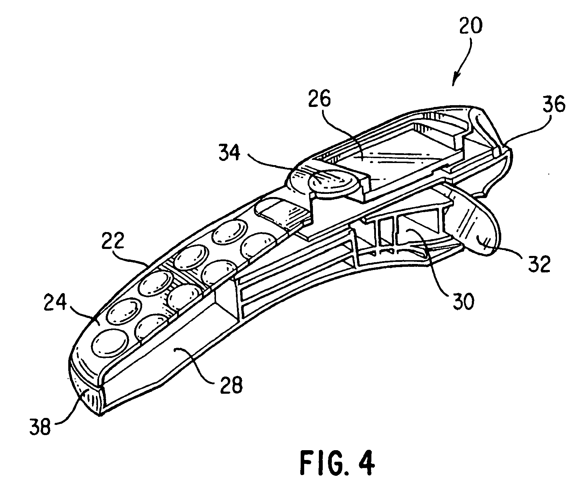

Referring to FIGS. 3 and 4, an analyte test instrument 20 includes a housing 22, a user interface 24, and a display area 26. The housing 22 is shaped to permit hand-held operation for bedside patient testing. The housing 22 includes an internal subframe 28 for mounting an analog and digital printed circuit boards and a barcode scan engine 30. The subframe also forms the battery cavity and includes battery contacts (not shown). The housing 22 is fabricated from rubber or plastic (e.g., ABS Polycarbonate). Smooth surfaces and a minimum of exposed fasteners and seams help to minimize areas which can collect foreign material and facilitates cleaning of the instrument. Silicone rubber pads are attached with adhesive to the bottom of the housing 22 to prevent skidding.

The user interface 24 includes a numeric keypad and function buttons to activate/deactivate power, select test or menu modes, edit entries, terminate entries, and activate a barcode reader as a substitute for manual numeric entry. All buttons in the user interface are fully sealed (e.g., using membrane switches). The keypad and barcode reader allow operators to enter a variety of data, including operator and patient identification (ID) numbers, strip control lot numbers, calibration codes, and to set other instrument parameters (e.g., date time, security intervals, display backlighting). The barcode reader is preferred for entry of test strip calibration data because it eliminates the need to visually verify a test strip code during each test.

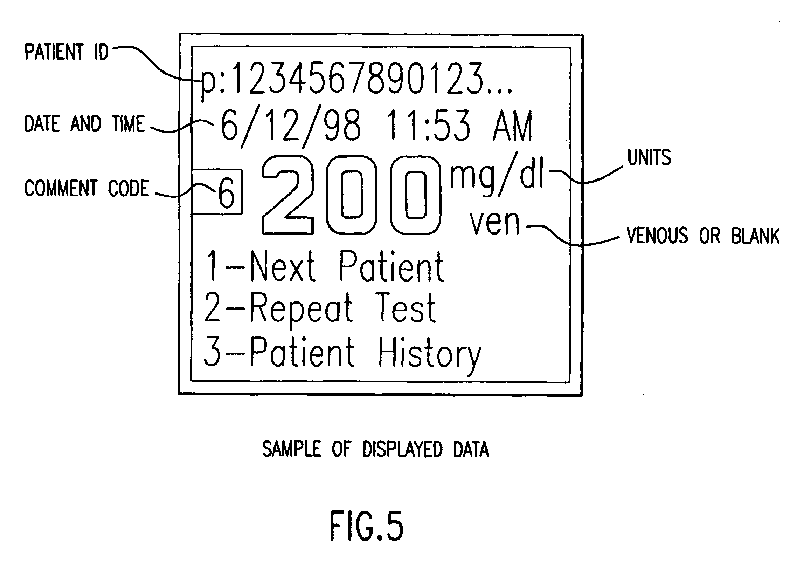

The display 26 is a graphic style liquid crystal display (LCD) module and provides multiple lines of text characters. Referring to FIG. 5, a variety of prompts, audio and visual warnings, and menu items can be displayed along with numerical test results. The display 26 includes a selectable backlight mode utilizing four amber high intensity LED's to improve visibility in poor lighting conditions.

Referring back to FIGS. 3 and 4, the barcode reader comprises a laser scan engine 30 and a red acrylic exit window 32. The red exit window 32 acts as an optical filter to reduce the received light that is not matched to the wavelength of the scan engine laser source (e.g., 680 nm). The barcode reader includes optics disposed at the top of the hand-held instrument to provide non-contact reading of barcodes. The reader is activated by depressing the scan key 34 located at the top of the keypad. The barcode reader can only be activated if the operator is prompted for entry of any one of the following: operator ID, patient ID, or strip lot or control vial information. Identification can be entered manually or read into the instrument via the reader from barcoded identification tags (e.g., wristbands) worn by operators and patients. Barcoded items placed within several inches of the exit window 32 can be scanned after depressing the scan button 34 in the user interface 24. An audible signal indicates successful reading of the barcode.

Barcode readers are well-known in the art (e.g., retail checkout scanners) and are commercially sold by Symbol Technologies, Inc. U.S. Patent No. 5,637,856, which is incorporated herein by reference, describes barcode scanning systems suitable for integration into an analyte instrument.

The instrument 20 includes a test strip port 36 which accepts test strips for determining the level of analyte in a sample taken from the patient. U.S. Patent No. 5,628,890, which is incorporated herein by reference, shows one type of test strip.

A data port ten pin connector 38 is provided in the base of the instrument to allow connection with mating contacts in the docking station for data transfer, battery recharge (from external power source), and printer communication. The connector does not extend beyond the contour of the base end of the instrument. A single row of electrical contacts within the connector is recessed to prevent inadvertent contact with external conductors. The instrument 20 responds to commands uploaded from the host computer linked through the data port. The external computer system initiates data transfer without any action on the part of the operator after the instrument has been mated to the docking station.

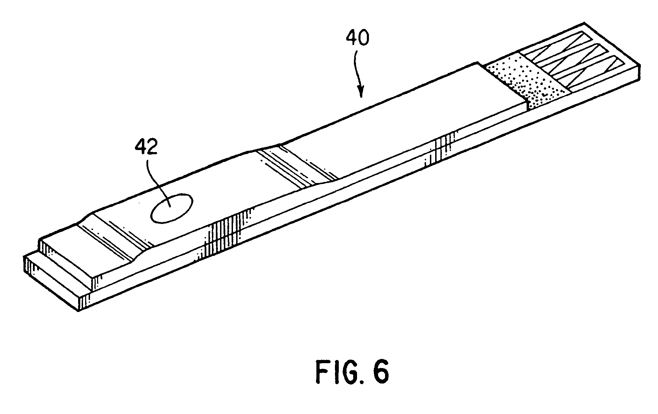

FIG. 6 shows one type of test strip 40 which includes three electrodes and can be used with the instrument (see FIG. 4) for determining the level of an analyte in the blood. The strip is partially inserted into the port 36 so that the sample area 42 remains outside the housing 22. The blood sample is applied to the sample area 42 and flows to an active area (not shown) at the unexposed ends of the three electrodes. The active area creates an electrochemical reaction in the sample, which is monitored electrically. Because each test strip typically has an expiration date, a strip identifier code which can be located on the strip package is either manually entered or scanned by the barcode reader into the instrument 20. If the strip code is not recognized as a valid code, then the instrument 20 alerts the operator and prevents further operation of the instrument 20 with that strip 40.

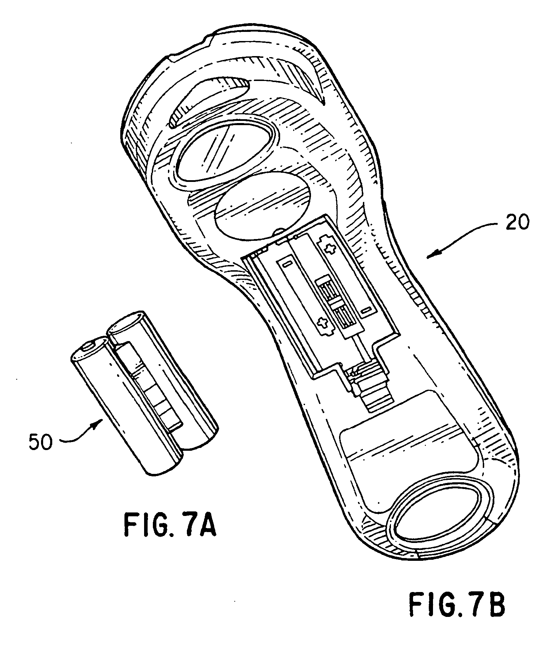

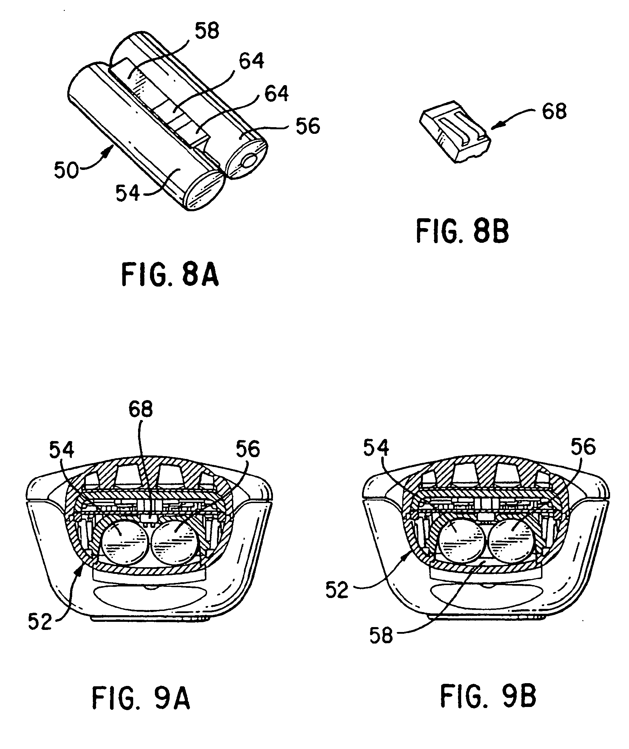

Referring to FIGS. 7 and 8A, the instrument 20 is powered by a rechargeable battery pack 50 (e.g., a nickel metal hydride (NiMH) battery package) securely disposed in a cavity 52 (i.e., battery compartment) on the underside of the instrument 20. The installed battery pack 50 (see FIG. 9A) is recharged by the docking station when the instrument is positioned in the docking station. Alternatively, the instrument can be powered by two standard alkaline batteries which are securely disposed in the same cavity 52 (see FIG. 9A). If an alkaline battery is installed improperly, it will not make electrical contact and the instrument will not turn on.

In addition, the possibility of inadvertently recharging the alkaline batteries is eliminated through the use of the custom-designed rechargeable battery pack 50. Also, keying features in the battery compartment are designed to prevent incorrect insertion of the battery pack or the insertion of a non-specified battery pack, thus eliminating the possibility of another battery chemistry from inadvertently being used.

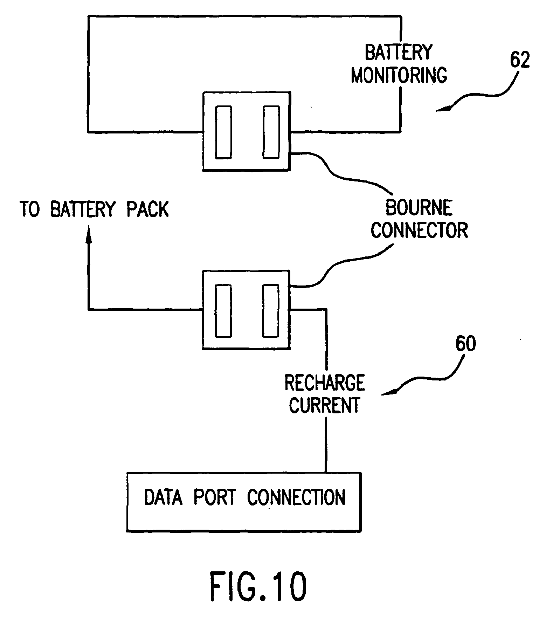

Referring to FIGS. 9A and 9B, the custom-designed battery pack 50 takes advantage of the void space that exists between two standard alkaline batteries 54, 56 when installed in the battery compartment 52, thereby eliminating the possibility of standard alkaline cells from activating the recharge functions. The recharge circuitry includes two independent circuits (see FIG. 10). The first circuit 60 provides recharge current to the rechargeable battery pack 50. The second circuit 62 determines the presence of the rechargeable battery pack 50 in order to facilitate the measuring of battery level. The pack 50 includes a plastic spine 58 which acts as a holder for the two NiMH batteries 54, 56 and occupies the void space which normally exists between two installed alkaline batteries. Referring back to FIG. 8A, two discrete conductive pads 64, 66 located in the plastic spine 58 act as bus bar contacts. Each bus bar contact is used in conjunction with a small two finger leaf spring contact connector 68 (e.g., Bourne connector) located within the void space in the battery compartment 52 (see FIGS. 8B and 9A - 9B). Each finger is electrically independent of the other finger in the connector. When the battery pack 50 is installed, the two electrically discrete bus bar contacts in the plastic spine 58 create an electrical short across each of the two connectors, thereby completing two independent circuits (see FIG. 10). Because completion of the electrical paths requires electrical current to flow from one contact on the connector, through the bus bar contact, and out the other contact of the same connector, there is no possibility that recharge current can be supplied in any other battery system which does not utilize this battery pack configuration.

### Docking Station

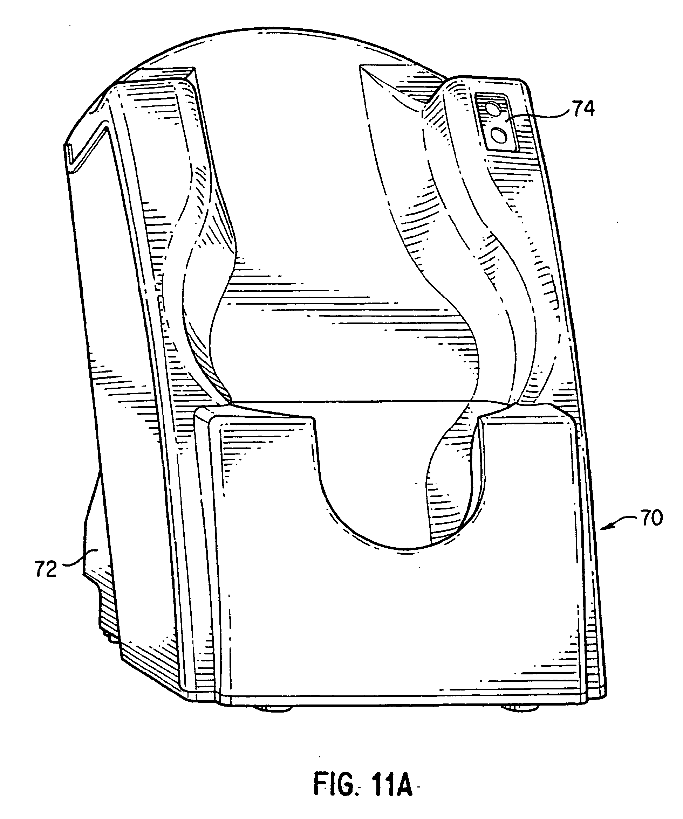

FIG. 11A shows a docking station 70 in a desk mount configuration. An alternate wall mount configuration is achieved by repositioning an attached mounting bracket 72. The docking station 70 provides at least the following two important capabilities for the instrument. First, an instrument with a rechargeable battery pack is recharged when seated in the docking station. Second, data communication with the host computer or other devices can be established through the docking station. In particular, the docking station is capable of hands-free and near real-time transfer of (1) test data to a host computer and (2) configuration data from the host computer. The docking station 70 also serves as a convenient resting place for the instrument when not in use.

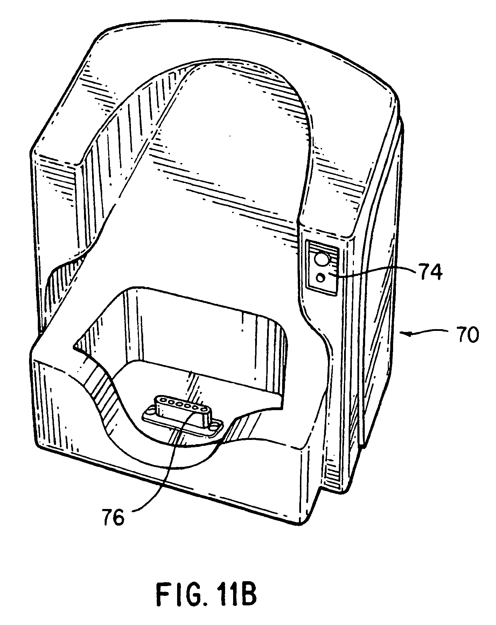

Power is provided to the docking station through an external AC adapter. Status lights 74 (e.g., LEDs) on the docking station indicate when power is on, when a meter has been docked successfully, and when data are being transferred through the docking station. The station 70 includes a docking connector 76 (see FIG. 11B) which includes a series of electrical contacts and is located in the recessed base. When the instrument is docked, the docking connector 76 receives a low insertion force (LIF) mated connector located in the base of the instrument. One of the station connector contacts provides power to the instrument for recharging the battery pack. The battery charge provided to the instrument is a low current (i.e., trickle charge) received from the docking station 70 through the instrument's data port connector 38. The docking station 70 incorporates circuitry to limit overcharging. Although the charging current is available at all times, only instruments equipped with a rechargeable battery pack are capable of receiving this current.

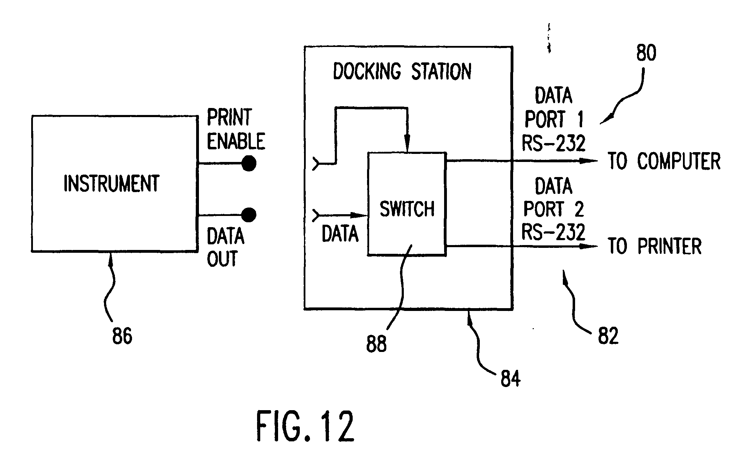

Referring to FIG. 12, the data connection through the docking station is essentially a pass-through connection from the instrument data port connector (see FIG. 4) to one of two standard 9-pin RS-232 ports. A first data port 80 is used for data transfer (e.g., to a computer, a modem, or an Ethernet terminal server) and the other port 82 is available for connection to a peripheral device (e.g., a printer). In its default condition, the docking station 84 is configured to pass data between the instrument 86 and the first data port 80. Data is passed to the second data port 82 when the docked instrument sets a switch 88 for the print mode. After data transfer through the second port 82 is completed, the docking station 84 resets switches to connect back to the first port 80.

The docking station 84 can be connected via a computer interface cable to a computer, a modem serial port, or some other communications port (e.g., Lantronix box) for data transfer over a communication line (e.g., a telephone or Ethernet TCP/IP line). The cable includes a standard nine pin RS-232 connector which mates with the docking station 84. A similar cable is used to communicate with a printer or other external device.

## Claims

1. A docking station (12,70,84) for receiving a hand-held analyte test instrument (10,86), the docking station comprising:
a connector (76) electrically connectable to **a data port connector (38) of** the instrument (10,86) for receiving analyte data therefrom **when said instrument (10,86) is seated and docked in a recessed base of the docking station (12,70,84);**
a switch (88) in electrical communication with the connector (76);
a first data port (80) in electrical communication with the switch (88) and being electrically connectable to a computer (14);
a second data port (82) in electrical communication with the switch (88) and being electrically connectable to a peripheral device (16);
a control mechanism for controlling the switch (88) to selectively pass the analyte data to the computer (14) via the first data port (80) or to the peripheral device (16) via the second data port (82);
said docking station (12,70,84) being configured to pass data between said analyte test intstrument (10,86) and said first data port (80) when said docking station (12,70,84) is in a default condition; and
**characterised by**
means to recharge the battery of the instrument, wherein the recharge is provided via said connector (76) **through said data port connector (38),** and said docking station (12,70,84) being further provided with a circuit to limit the overcharging.

2. A docking station (12,70,84) according to claim 1 in a desk mounted configuration.

3. A docking station (12,70,84) according to claim 1 in a wall mounted configuration.

4. A, docking station according to any of the preceding claims, said docking station (12,70,84) being further provided with status lights (74).

5. A docking station (12, 70, 84) according to any of the preceding claims wherein the peripheral device is a printer.

6. A docking station (12, 70, 84) according to any of the preceding claims which allows the passage of data from the instrument (10,86) to computer (14), a modem serial port or other communications port.

## Patentansprüche

1. Eine Docking Station (12,70,84) zur Aufnahme eines tragbaren Analysegeräts (10,86), wobei die Docking Station folgendes umfaßt:
einen Anschluß (76), der elektrisch anschließbar ist an einen Dateneingangs-Anschluß (38) des Instruments (10,86) zum Empfangen von Analysedaten davon, wenn das Instrument (10,86) in eine vertiefte Basis der Docking Station (12,70,84) gelegt und angedockt wird;
einen Schalter (88) in elektrischer Verbindung mit dem Anschluß (76);
einen ersten Dateneingang (80) in elektrischer Verbindung mit dem Schalter (88) und welcher elektrisch an einen Computer (14) anschließbar ist;
einen zweiten Dateneingang (82) in elektrischer Verbindung mit dem Schalter (88) und welcher an eine Peripherievorrichtung (16) elektrisch anschließbar ist;
einen Steuermechanismus zur Steuerung des Schalters (88), um die Analysedaten selektiv an den Computer (14) über den ersten Dateneingang (80), oder zu der peripheren Vorrichtung (16) über den zweiten Dateneingang (82) zu übertragen;
wobei die Docking Station (12,70,84) so konfiguriert ist, um Daten zwischen dem Analysengerät (10,86) und den ersten Dateneingang (80) zu übertragen, wenn die Docking Station (12,70,84) in einem Grundzutand ist; und
**gekennzeichnet durch** Mittel, um die Batterie des Instruments wieder aufzuladen, wobei die Wiederaufladung über den Anschluß (76) **durch** den Dateneingangsanschluß (38) bereitgestellt wird, und wobei die Docking Station (12,70,84) weiter mit einer Schaltung ausgestattet ist, um die Überladung zu begrenzen.

2. Eine Docking Station (12,70,84) gemäß Anspruch 1, in einer Tisch-montierten Anordnung.

3. Eine Docking Station (12,70,84) gemäß Anspruch 1 in einer Wand-montierten Anordnung.

4. Eine Docking Station gemäß irgendeinem der vorhergehenden Ansprüche, wobei die Docking Station (12,70,84) weiter mit Zustandslichtern (74) ausgestattet ist.

5. Eine Docking Station (12,70,84) gemäß irgendeinem der vorhergehenden Ansprüche, worin die Peripherievorrichtung ein Drucker ist.

6. Eine Docking Station (12,70,84) gemäß irgendeinem der vorhergehenden Ansprüche, welche den Durchgang von Daten von dem Instrument (10,86) zu dem Computer (14), einem seriellen Modemanschluß oder einem anderen Verbindungsanschluß, erlaubt.

## Revendications

1. Station d'accueil (12, 70, 84), pour recevoir un instrument portatif de test d'analyte (10, 86), la station d'accueil comprenant :
un connecteur (76), susceptible d'être connecté électriquement à un **connecteur de port de données (38)** de l'instrument (10, 86) pour en recevoir des données d'analyte, **lorsque ledit instrument (10, 86) est mis en place et installé dans une base en creux de la station d'accueil (12, 70, 84) ;**
un interrupteur (88), en communication électrique avec le connecteur (76) ;
un premier port de données (80), en connexion électrique avec l'interrupteur (88) et susceptible d'être connecté électriquement à un ordinateur (14) ;
un deuxième port de données (82), en connexion électrique avec l'interrupteur (88) et susceptible d'être connecté électriquement à un dispositif périphérique (16) ;
un mécanisme de commande, pour commander l'interrupteur (88) afin de transmettre sélectivement les données d'analyte à l'ordinateur (14), via le premier port de données (80), ou au dispositif périphérique (16), via le deuxième port de données (82) ;
ladite station d'accueil (12, 70, 84) étant configurée pour laisser passer des données entre ledit instrument de test d'analyte (10, 86) et ledit premier port de données (80), lorsque ladite station d'accueil (12, 70, 84) est en un état par défaut ; et
**caractérisé par** des moyens pour recharger la batterie de l'instrument, dans lequel la recharge est effectuée via ledit connecteur (76), **par l'intermédiaire dudit connecteur de port de données (38),** et ladite station d'accueil (12, 70, 84) étant en outre muni d'un circuit de limitation de surcharge.

2. Station d'accueil (12, 70, 84) selon la revendication 1, dans une configuration montée sur un bureau.

3. Station d'accueil (12, 70, 84) selon la revendication 1, dans une configuration montée sur un mur.

4. Station d'accueil selon quelconque l'une des revendications précédentes, ladite station d'accueil (12, 70, 84) étant en outre muni d'indicateurs lumineux d'état (74).

5. Station d'accueil (12, 70, 84) selon la revendication 1, dans lequel le dispositif périphérique est une imprimante.

6. Station d'accueil (12, 70, 84) selon quelconque l'une des revendications précédentes, permettant le passage de données, de l'instrument (10, 86) à l'ordinateur (14), un port série de modem ou un autre port de communication.
